# EUROPEAN PATENT SPECIFICATION

(11) **EP 3 285 703 B1**
(45) Date of publication and mention of the grant of the patent: **09.01.2019**
(21) Application number: 15790663.7
(22) Date of filing: 11.09.2015
(51) Int. Cl.: A61F 5/44, A61F 5/441

(54) **SECONDARY BAG FOR URINE**
SEKUNDÄRBEUTEL FÜR URIN
SAC SECONDAIRE POUR L'URINE

(30) Priority: 23.04.2015 IT PE20150009
(43) Date of publication of application: 28.02.2018
(73) Proprietor: Evoluzione S.r.l., 00144 Roma (IT); Servimed S.r.l., 00144 Roma (IT); Folcando, Antonio, 71121 Foggia (IT); Di Teodoro, Alessandro, 65124 Pescara (PE) (IT)
(72) Inventor: FOLCANDO, Ettore, I-65100 Pescara (IT); NAPOLITANO, Francesco, I-00144 Roma (IT); DE LUTIO, Enrico, I-00144 Roma (IT); FOLCANDO, Antonio, 71121 Foggia FG (IT); DI TEODORO, Alessandro, 65124 Pescara (IT)
(74) Representative: Di Giovine, Paolo
(86) International application number: PCT/IB2015/056965
(87) International publication number: WO 2016/170403

(56) References cited:
- FR-A1- 2 501 499
- GB-A- 2 309 906
- US-A- 4 265 118
- US-A- 5 354 132
- US-A1- 2010 152 686

## Description

### Field of the invention

The present invention relates to a bag apt to be connected to the primary bags in turn connected to a catheter so as to perform safely the emptying of the bag, by eliminating all risks connected to the accidental spilling of the liquids.

### State of art

Currently in the hospitalization days many patients are catheterized, that is they are provided with a small tube made of lattice or silicone which is introduced into the bladder through urethra to favour the discharge of urine outside. The main problem linked to the use of catheters is that the bags connected to the catheter, once they have filled up, have to be emptied to be disposed of.

The patent application FR2501499 discloses a secondary bag for urine apt to connect to a primary bag.

The risks connected to the handling of such bags, in particular in the emptying step, force the operators to provide themselves with personal protection equipment to lower the infection risk during this process.

The object of the present invention is to solve this problem by providing a bag for urine according to claim 1.

Additional features of the bag for urine of the subject invention are defined in the corresponding depending claims.

The present invention, by overcoming the mentioned problems of the known art, involves several and evident advantages:
removal of contamination risk for the operator caused by splashes during emptying, since such procedure is no more necessary thanks to the fact that the bag is disposed of as solid waste. Consequently, the operator needs no more to provide himself/herself with personal protection equipment as he/she acts under maximum safety conditions.

The sachet of water-soluble material existing in the bag, as soon as it comes in contact with urine, dissolves quickly by allowing the molecule super absorbent to transform into solid substance the urine coming from the main bag. In this way it is avoided that the content of the same remains liquid and then more subjected to risks of accidental spillage.

To support what mentioned above, an additional thing to be added is that the waste produced after gelification is by now a solid waste, therefore less onerous to be handled and above all less dangerous (as without risks due to possible splashes). Other advantages, as well as the features and the use modes of the present invention will result evident from the following detailed description of some preferred embodiments, shown by way of example and not for limitative purposes, by making reference to the figures of the enclosed drawings, wherein:
figure 1 is an overall view of the bag according to a preferred embodiment of the present invention.

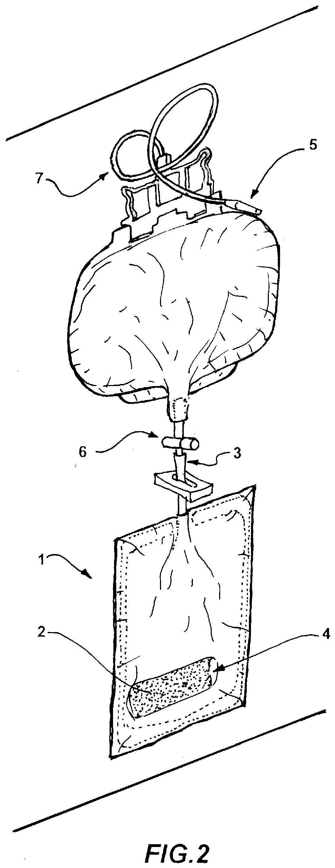

figure 2 is an overall view of a kit used according to a preferred embodiment of the present invention.

The present invention will be described hereinafter in details by making reference to the above-mentioned figures.

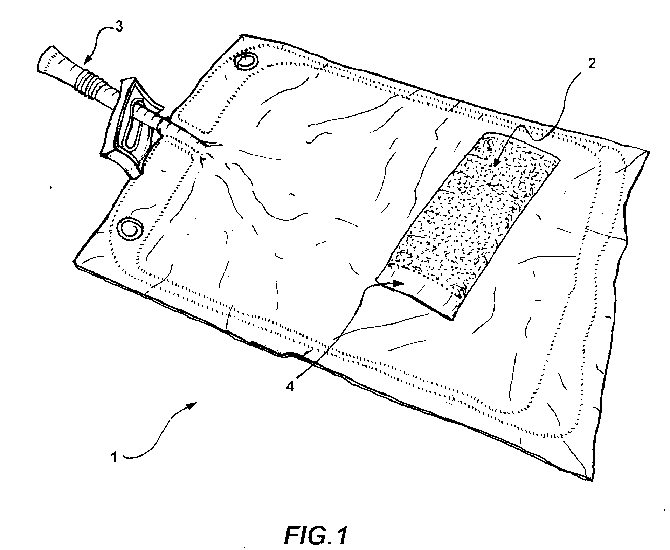

By firstly making reference to figure 1, this represents an overall view showing, as a whole, a bag 1 according to the present invention, according to a preferred embodiment.

The secondary bag of the present invention is a bag suitable to include liquid organic waste, in particular urine, and it is characterized in that it includes inside thereof a water soluble bag 4 comprising inside thereof a substance 2 apt to absorb urine. The secondary bag 1 further comprises means 3 apt to connect said secondary bag 1 to a primary bag 5 whereby the urine contained in said primary bag 5 is emptied into said secondary bag 1.

In the present description under the term primary bag 5 any bag is meant suitable to include liquid organic waste, in particular urine, connected or connectable to a catheter 7 and comprising means 6 for emptying the primary bag, for example standard connectors known to the person filled in the art currently used for emptying this type of bags.

In the present description under the term secondary bag 1 any bag is meant suitable to include liquid organic waste, in particular urine, comprising means 3 apt to connect said secondary bag 1 to a primary bag 5 whereby when the primary bag 5 is filled up with urine, the urine included in the primary bag 5 can be emptied into the secondary bag 1. Said means 3, for example, comprises a universal connector connectable to means 6 for emptying the primary bags 5.

Example of substances suitable to absorb the urine are super absorbent powders with absorption capacity between 60 and 500 times the weight of the liquids, such as for example the cross-linked sodium polyacrylate, preferably provided with system aromatizer, for example the one available under the trade name of *Sgaiel.* Such powders are able to transform the urine into gel. Under provided with "system aromatizer" it is meant that the substance absorbent can be itself perfumed or be mixed to an additional perfumed substance.

A subject of the present invention is even a kit comprising one or more secondary bags according to the present invention and at least a primary bag 5. According to an embodiment, such kit comprises one or more catheters 7 connected or connectable to said primary bag 5. Preferably the catheter 7 will be connected to the primary bag 5 in opposite position with respect to the emptying means 6.

In the light of the above-illustrated description it appears clear that the operation of the bag and of the subject kit is extremely easy as it is sufficient that the operator connects the secondary bag to the primary bag which he/she has to empty and during the filling-up thereof the molecule will enter in function automatically thanks to the melting of the water-soluble sack.

The present invention has been sofar described by referring to some embodiments thereof. It is to be meant that other embodiments belonging to the same inventive core may exist, all within the protective scope of the herebelow reported claims.

## Claims

1. A secondary bag (1) for urine comprising means (3) apt to connect said secondary bag (1) to a primary bag (5) whereby the urine contained in said primary bag (5) is emptied into said secondary bag (1) and wherein said secondary bag (1) is **characterized in that** containing a water soluble bag (4) containing a substance (2) apt to absorb urine, wherein said substance (3) is a molecule absorbent adapted to transform the urine into gel.

2. The bag (1) according to claim 1, wherein said substance is a powder absorbent with absorption capacity between 60 and 500 times the weight of the liquid to be absorbed.

3. The bag (1) according to any one of claims 1 to 2 wherein said substance (2) is the cross-linked sodium polycrylate.

4. The bag (1) according to any one of claims 1 to 3, wherein said substance (2) further comprises a system aromatizer.

5. The bag (1) according to any one of claims 1 to 4, made in polyethylene, biodegradable polyethylene or pvc.

6. The bag (1) according to any one of claims 1 to 5, wherein said means (3) comprise a universal connector connectable to the emptying means (6) of said primary bag (5).

7. The bag (1) according to any one of claims 1 to 6, wherein said primary bag (5) is filled with urine via a catheter (7) connected or connectable to said primary bag (5).

8. A Kit comprising one or more bags (1) according to any one of the preceding claims and at least one primary bag (5).

9. The Kit according to claim 8 further comprising one or more catheters (7) connected or connectable to said primary bag (5).

## Patentansprüche

1. Sekundäre Tasche (1) für Urin mit Mitteln (3), die geeignet sind, die sekundäre Tasche (1) mit einer primären Tasche (5) zu verbinden, wobei das in der primären Tasche (5) enthaltene Urin in die sekundäre Tasche (1) entleert wird und wobei die sekundäre Tasche (1) **dadurch gekennzeichnet ist, dass** sie eine wasserlösliche Tasche (4) enthält, die eine Substanz (2) enthält, welche geeignet ist, Urin zu absorbieren, wobei die Substanz (3) ein molekulares Absorbens ist, das geeignet ist, den Urin in Gel zu transformieren.

2. Tasche (1) nach Anspruch 1, wobei die Substanz ein Puderabsorbens mit einer Absorptionskapazität zwischen 60- und 500-mal dem Gewicht der zu absorbierenden Flüssigkeit ist.

3. Tasche (1) nach einem der Ansprüche 1 bis 2, wobei die Substanz (2) quervernetztes Natriumpolyacrylat ist.

4. Tasche (1) nach einem der Ansprüche 1 bis 3, wobei die Substanz (2) weiterhin einen Systemaromatisierer umfasst.

5. Tasche (1) nach einem der Ansprüche 1 bis 4, gefertigt aus Polyethylen, biodegradierbarem Polyethylen oder PVC.

6. Tasche (1) nach einem der Ansprüche 1 bis 5, wobei die Mittel (3) einen universellen Verbinder umfassen, der mit den Entleerungsmitteln (6) der primären Tasche (5) verbindbar ist.

7. Tasche (1) nach einem der Ansprüche 1 bis 6, wobei die primäre Tasche (5) mit Urin über einen Katheter (7) befüllt wird, der mit der primären Tasche (5) verbunden oder verbindbar ist.

8. Kit mit einer oder mehreren Taschen (1) nach einem der vorhergehenden Ansprüche und wenigstens einer primären Tasche (5).

9. Kit nach Anspruch 8, das weiterhin einen oder mehrere Katheter (7) umfasst, die mit der primären Tasche (5) verbunden oder verbindbar sind.

## Revendications

1. Sac secondaire (1) pour urine comprenant des moyens (3) aptes à raccorder ledit sac secondaire (1) à un sac principal (5), moyennant quoi l'urine contenue dans ledit sac principal (5) est vidée dans ledit sac secondaire (1) et dans lequel ledit sac secondaire (1) est **caractérisé en ce qu'**il contient un sac soluble dans l'eau (4) contenant une substance (2) apte à absorber l'urine, dans lequel ladite substance (3) est un agent d'absorption moléculaire adapté pour transformer l'urine en gel.

2. Sac (1) selon la revendication 1, dans lequel ladite substance est un agent d'absorption en poudre avec une capacité d'absorption comprise entre 60 et 500 fois le poids du liquide à absorber.

3. Sac (1) selon l'une quelconque des revendications 1 à 2, dans lequel ladite substance (2) est du polyacrylate de sodium réticulé.

4. Sac (1) selon l'une quelconque des revendications 1 à 3, dans lequel ladite substance (2) comprend en outre un aromatiseur de système.

5. Sac (1) selon l'une quelconque des revendications 1 à 4, réalisé en polyéthylène, polyéthylène biodégradable ou PVC.

6. Sac (1) selon l'une quelconque des revendications 1 à 5, dans lequel lesdits moyens (3) comprennent un connecteur universel pouvant être raccordé aux moyens de vidage (6) dudit sac principal (5).

7. Sac (1) selon l'une quelconque des revendications 1 à 6, dans lequel ledit sac principal (5) est rempli avec l'urine via un cathéter (7) raccordé ou pouvant être raccordé audit sac principal (5).

8. Kit comprenant un ou plusieurs sacs (1) selon l'une quelconque des revendications précédentes et au moins un sac principal (5).

9. Kit selon la revendication 8, comprenant en outre un ou plusieurs cathéters (7) raccordés ou pouvant être raccordés audit sac principal (5).
